# EUROPEAN PATENT APPLICATION

(11) **EP 2 239 333 A1**
(43) Date of publication of application: **13.10.2010**
(21) Application number: 09157525.8
(22) Date of filing: 07.04.2009
(51) Int. Cl.: C12P 3/00, C12P 7/46, C12P 7/52, C12P 7/54, C12P 7/56

(54) **Processes for recovery of organic acids from aqueous solutions obtained from bio-organic materials**

(71) Applicant: Sanovations B.V., 9722 WL Groningen (NL)
(72) Inventor: Sanders, Constantijn Ferdinand Willem, 9722 WL, Groningen (NL); Sanders, Johan Pieter Marinus, 9722 WL, Groningen (NL)
(74) Representative: Swinkels, Bart Willem

(57) **Abstract**

The present invention relates to processes for recovery of components from bio- organic juice streams using ion exchange. The process includes a fermentation step wherein non-charged organic molecules such as sugars are converted to organic acids, such as lactic acid, that may be recovered by ion exchange. The process further includes an optional step wherein the ion exchangers are regenerated using gaseous ammonia, which is preferably obtained from calcium hydroxide treatment of lignocellulosic material, such as e.g. a biogas digestate.

## Description

### Field of the invention

The invention relates to the recovery of components from juice stream and other aqueous solutions obtained from bio-organic, e.g. vegetable or fermented/fermentation materials.

### Background of the invention

For recovery of components from vegetable materials various techniques are known in the art, most of which include mechanical steps for breaking up the plant material, which may then be fractionated into at least two fractions, a solid fraction containing e.g. insoluble fibres and a liquid fraction such as e.g. a juice stream, containing most of the nutritionally valuable components. Techniques used for recovery of components from vegetable juice streams include e.g. extraction, evaporation, membrane-filtration, crystallisation, and heat coagulation of e.g. sugars or starch or proteins. However, a large part of soluble plant components may not be easily and efficiently recovered because of the low concentrations in which they are present.

Many of these techniques require significant amounts of capital and/or energy and/or other operational cost such as chemicals, membranes and labour costs, preventing cost-effective recovery of the components. E.g. extraction of components present in low concentrations may require successive rounds of extraction and subsequent evaporation for concentration of the components. Evaporation generally is a very energy-intensive method for concentration of components. Moreover, the higher temperatures required for evaporation may result in loss of the component to be recovered due to thermal instability. Even though membranes may be used to filter molecules of a specific size from a solution, large amounts of energy are required for pressing the solvent through the membrane.

It is an object of the present invention to address these problems and to provide for processes for the recovery of components from aqueous solutions obtained from bio-organic (e.g. vegetable) materials that are energy efficient and with a minimised burden to the environment.

### Description of the invention

In a first aspect the invention relates to a process for recovery of components from a bio-organic juice stream using ion exchange.

A "bio-organic juice stream" is herein broadly understood to be any liquid composition comprising biological and organic matter of plant, animal or microbial origin (including mixtures thereof). The "bio-organic juice stream" will usually be an aqueous solution comprising plant-, animal- and/or micro-organism-derived components, including e.g. organic acids and/or mineral acids. A preferred juice stream for use in the present invention is a "vegetable juice stream", which is herein broadly understood to be any liquid composition comprising plant-derived components. The juice stream will usually be an aqueous solution comprising plant-derived components. Other bio-organic juice streams may be obtained from fermented/fermentation materials, e.g. obtained by fermentation of plant material but also by fermentation of animal material such as offal, manure, or milk.

In general, in the context of the present invention, raw materials, such as e.g. vegetable raw materials, are usually first mechanically processed by e.g. chopping, grinding, milling, mashing, crunching and pressing to break up, fragmentise and/or comminute the plant materials. Next or before the mechanical step a heat treatment can be included to open up the plant cells or the open up the fibrous materials in the presence or absence of added chemicals such as organic acids or mineral acids. Next, the raw material may be separated into a solid (press cake) fraction, comprising most of the solids, such as fibres in the raw material, and a liquid juice fraction. Methods for separating vegetable material, such as e.g. grasses, lucerne, potato tops (i.e. stems and leafs) pea tops and beet tops, into a solid fibre fraction and a juice stream are disclosed in EP 1 141 450.

The vegetable juice will usually consists substantially of the aqueous content of plant cells, i.e. the vacuole content and the cytoplasm having therein cell organelles such as chloroplasts in intact or disintegrated form. Cell wall constituents (fibres) will usually be substantially absent from the juice in that they remain behind in the press cake. The vegetable juice stream as contemplated by the invention may thus consists of an aqueous solution/suspension of virtually all high-grade components or nutrients from the vegetable material such as soluble starch, sugars, fructose-oligosaccharides, proteins, amino acids, vitamins, fertilizers such as phosphate, lipids, pigments, organic acids, naturally occurring and/or by GMO chemical building blocks and the like. It is further understood that the solid fraction or press cake may be further extracted by washing or rinsing one or more times. The washings thus obtained may be used as such as vegetable juice stream in the processes of the invention or they may be combined with the initial juice stream to be further processed in accordance with the invention. The colour of the defibered juice stream can typically be green due to the presence of intact or broken chloroplasts, sometimes brown-green through browning during the fractionation. Macroscopically, the juice is a liquid. Microscopically, principally intact and disintegrated parenchyma cells and cell organelles such as chloroplasts are visible in this liquid.

The juice stream for use in a process according to the invention may in principle be derived from any vegetable material, originating both from cultivated plants (crop plants) and from wild plants, as well as mixtures of plants. Examples include e.g. vegetable biomass originating from cultivated grassland, but also from natural grounds and roadsides (weeds), feed crops such as forage grasses, corn, lucerne, clover, spinach, papilionaceous plants, grains, beets, peas, beans, potatoes, carrots, cassava, sweet potato, sugarcane, algae, soy and fibre crops such as flax and hemp juice, as well as juice from industrial streams such as soy milk production and other diluted streams. Grass, lucerne and other fresh and green harvested plants which, often as virtually whole plant, in particular the leaf and/or stem parts and in most cases not including the roots, (e.g. the tops of crops normally grown solely for their seeds, fruits or tubers, such as grains, beets, peas, beans, potatoes, carrots, cassava, sweet potato) may be used in the processes of the invention. Further juice streams to be used in the processes of the invention include juice streams obtained from industrially processed parts of plants such as potato, beet, cane, wheat, corn, soy, rape and others, including juice streams obtained as waste streams resulting from such processes, such as e.g. potato fruit water (potato waste water). In a preferred process of the invention the juice is made from fresh or conserved grass. A vegetable juice stream may be obtained from such vegetable raw materials through pressing of (preferably chopped or otherwise comminuted) leaf and/or stem material, whereby a part of the vegetable material is obtained as press juice, while the residual and pressed material is known as press cake.

The use of ion exchange for recovery of components from juice streams is particularly advantageous for recovery of components present in low concentrations. A component present in a low concentration is herein understood as an individual component being present at a concentration of less than 5, 2, 1, 0.5, 0.2 or 0.1% (w/v) in the juice stream or in the untreated or unfermented juice stream. Non-charged organic compounds present in the juice at low concentrations may first be converted to organic acids by fermentation in accordance with the second aspect of the invention (see below), to render them amenable to recovery by ion exchange.

In one embodiment a process of the invention comprises the step of binding components in the juice stream to an ion exchanger. Thus the juice stream is brought into contact with the ion exchanger. The ion exchanger preferably is a solid ion exchanger such as solid polymeric or mineralic ion exchangers. A preferred ion exchanger is an ion exchange resin, which is preferably in the form of beads. Typical ion exchangers are ion exchange resins (functionalized porous or gel polymer), zeolites, montmorillonite, clay, and soil humus. Ion exchangers for use in the present invention may either be cation exchangers that exchange positively charged ions (cations) or anion exchangers that exchange negatively charged ions (anions). Also mixed ion exchangers, i.e. composed of at least one cation exchanger and at least one anion exchanger, can be applied.

In one embodiment, the juice stream is brought into contact with ion exchanger by applying the juice to a column comprising the ion exchanger. A preferred embodiment is in a vertical position and the direction of the flow of the juice stream through the ion exchanger (in e.g. a column) is (essentially) against the gravity. E.g. when the column is in an essentially vertical position, the juice stream enters at the bottom and leaves at the top of the column. To prevent ion exchange material from leaving the column, a mesh be present at the exit of the column and/or the flow rate of the juice through the column may be such that gravity prevents ion exchange material from leaving the column.

Preferably the process of the invention at least comprises a step wherein anions in the juice stream are bound to an anion exchanger. Subsequently, the anion exchanger (comprising the bound anion from the juice) is separated from the juice stream, optionally washed, and then the anions are eluted from the anion exchanger and the anion exchanger is regenerated. In the process of the invention the juice may be subjected to more than one round anion exchange, wherein the order of the different rounds of anion exchange, the type of anion exchanger and the conditions of anion exchange (including elution and regeneration) are determined by the type of anions to be recovered from the juice stream as described herein below.

In a second aspect, the invention relates to a process for recovery of components from a juice stream using ion exchange, wherein the process comprises a step wherein organic compounds in the juice stream are fermented to organic acids. Preferably in this aspect, the process further comprises a step wherein the organic acids are recovered from the (fermented) juice stream using an anion exchanger.

The organic compounds in the juice stream preferably include non-charged organic compounds and other organic compounds that are not easily recovered from the juice using ion exchange. Organic compounds in the juice stream to be fermented to organic acids may be any organic compound that can serve as a carbon and/or energy source for a micro organism that produces organic acids or that is capable of producing organic acids. The organic compounds in the juice stream to be fermented to organic acids may comprise one or more of carbohydrates, in particular sugars like mono-, di-, oligo- and/or polymers comprising one or more of glucose, fructose, galactose, xylose, arabinose, saccharose, maltose, trehalose, and maltodextrine, as well as lipids.

Preferably in this aspect, the organic compounds in the juice stream are fermented to produce at least one organic acid selected from the group consisting of lactic acid, acetic acid, propionic acid, butyric acid, valeric acid, citric acid, fumaric acid, xylonic acid, gluconic acid, glucuronic acid, succinic acid, tartaric acid, malic acid, maleic acid, malonic acid, ascorbic acid, chorismic acid, glucaric acid, glutaric acid, adipic acid, oxalic acid, formic acid, itaconic acid, levulinic acid, aconitic acid, ascorbic acid, kojic acid, and coumaric acid.

The fermentation process for producing organic acids from the organic compounds in the juice stream may be an aerobic or an anaerobic fermentation process. An anaerobic fermentation process is herein defined as a fermentation process run in the absence of oxygen or in which substantially no oxygen is consumed, preferably less than 5, 2.5 or 1 mmol/L/h, more preferably 0 mmol/L/h is consumed (i.e. oxygen consumption is not detectable), and wherein organic molecules serve as both electron donor and electron acceptors. In the absence of oxygen, NADH produced in glycolysis and biomass formation, cannot be oxidised by oxidative phosphorylation. To solve this problem many micro organisms use pyruvate or one of its derivatives as an electron and hydrogen acceptor thereby regenerating NAD⁺. Thus, in a preferred anaerobic fermentation process pyruvate is used as an electron (and hydrogen acceptor) and is reduced to organic acids as herein defined above. Anaerobic processes of the invention are preferred over aerobic processes because anaerobic processes do not require investments and energy for aeration and in addition, anaerobic processes produce higher product yields than aerobic processes. Alternatively, the fermentation process of the invention may be run under aerobic oxygen-limited conditions. Preferably, in an aerobic process under oxygen-limited conditions, the rate of oxygen consumption is at least 5.5, more preferably at least 6 and even more preferably at least 7 mmol/L/h.

The fermentation process may run at environmental temperatures, e.g. at a temperature of around 20, 22, or 25°C. To avoid contamination with other micro organisms the fermentation process may however advantageously be run at higher temperatures, e.g. at a temperature which is higher than 35, 37, 42, 45, 50, 55 or 60°C.

The fermentation process is preferably run at a pH in a range that is optimal for the fermenting micro organism. Optimal pH ranges for the various micro organism mentioned below are well known in the art and will usually be in the range of pH 4.0 to 8.0, e.g. pH 4.0 to 6.0, pH 5.0 to 7.0, pH 6.0 to 8.0 or narrower ranges such as pH 5.0, 6.0, or 7.0, each plus or minus 0.5 pH unit. The pH of the fermentation process may be controlled by addition of appropriate base such as e.g. NaOH or ammonia. Preferably, the (gaseous) ammonia is obtained from calcium hydroxide treatment of a lignocellulosic material as herein defined below.

In a preferred embodiment of this aspect of the invention, the organic compounds in the juice stream are fermented to organic acids that at least include lactic acid. For this purpose lactic acid-forming bacteria, yeast or fungi, or combinations of more than one lactic acid-forming micro organism, may be used. Suitable lactic acid producer organisms include natural and/or selected micro organisms or micro organisms produced by adaptation or mutated to produce a desired lactic acid. Lactic acid producer organisms include lactic acid bacteria, such as those of the genera *Lactobacillus*, *Leuconostoc*, *Pediococcus*, *Lactococcus*, and *Streptococcus* as well as the more peripheral *Aerococcus*, *Carnobacterium*, *Enterococcus*, *Oenococcus*, *Sporolactobacillus*, *Teragenococcus*, *Vagococcus*, and *Weisella*; all of which belong to the order *Lactobacillales* but also lactic acid-forming bacteria from the genera *Bacillus*, *Erysipelothrix*, *Gemella*, and *Globicatella.* Particularly preferred are thermophilic lactic acid forming bacteria such e.g. *Bacillus stearothermophilus* and the thermophilic lactic acid bacteria *Lb. helveticus, Lb. Delbrueckii, S. thermophilus* and *L. bulgaricus.* Eukaryotic lactic acid-producing micro organisms include yeasts from the genera *Kluyveromyces*, *Saccharomyces*, *Torulaspora* and *Zygosaccharomyces* and metabolically modified strains thereof (see e.g. US 7,326,550) and fungi from the genus *Rhizopus*, such as *Rhizopus oryzae.* The organic compounds in the juice stream may also be fermented to organic acids such as fumaric acid and/or itaconic acid using fumaric acid producing micro organisms including fungi from the genera *Rhizopus*, or *Aspergillus*, such as *Rhizopus oryzae* or *Aspergillus niger*, or itaconic acid producing micro organisms including fungi from the genus *Aspergillus terreus.*

In a preferred process of the invention, the organic acids produced in accordance with the second aspect of the invention in the (fermented) juice stream, are recovered by anion exchange.

In one embodiment the organic acids (and other anions including e.g. phosphate and/or amino acids) in the fermented juice stream are recovered by anion exchange without prior neutralisation of pH. Due to the production of the organic acids in the juice stream, the pH of the fermented juice stream will be acidic, e.g. below 6.0, 5.5, 5.0 or 4.5. A suitable anion exchanger for use in this embodiment is an anion exchanger that binds organic acids under acidic conditions. Examples of such anion exchangers include strong or weak basic resins. Strong basic resins include e.g. resins comprising quaternary amino groups such as trimethylammonium groups of which poly (acrylamido-N-propyltrimethylammonium chloride) (polyAPTAC). Commercially available examples of suitable strong basic resins include e.g. Amberlyst A21, Amberlyst A23, Sybron AFP-329, DOW Marathon WBA, Purolite A-100, ResinTech WBMP. Weak basic resins include e.g. resins comprising primary, secondary, and/or ternary amino groups, such as e.g. polyethylene amine. Commercially available examples of suitable weak basic resins include e.g. Amberlite IRA400, Amberlite IRA410, Sybron ASB-1, DOW SBR, Purolite A-600, ResinTech SBG1.

In another embodiment, the organic acids (and other anions including e.g. phosphate and/or amino acids) in the fermented juice stream are recovered by anion exchange after increasing the pH of the fermented juice stream. Preferably the pH of the fermented juice stream is increased using ammonia, of which gaseous ammonia is more preferred. Most preferred is gaseous ammonia obtained from calcium hydroxide treatment of a lignocellulosic material as herein defined below. The pH of the fermented juice stream is preferably increased to at least neutral pH, i.e. pH 6.0 to 8.0 or around pH 7.0, more preferably the pH is increased to alkaline pH, i.e. a pH higher than pH 8.0, 8.5, 9.0, 9.5. or 10.0. A suitable anion exchanger for use in this embodiment is an anion exchanger that binds organic acids under neutral and/or alkaline conditions. Examples of such anion exchangers include weak basic resins as described above.

In one embodiment, the process of the invention includes a step wherein, the juice stream is subjected to anion exchange at neutral pH or (mild) acidic pH prior to or without fermentation of the juice stream. Anion exchange of the untreated juice at neutral or acidic pH, i.e. pH 4.0 to 8.0, preferably pH 5.0 to 7.5, e.g. around pH 6.0, 6.5 or 7.0, may be used to recover acidic amino acids like glutamic acid and aspartic acid as well as organic acids and other anions already present in the juice stream such as e.g. citric acid, and inorganic anions such as chloride, sulphate, phosphate. A suitable anion exchanger for use in this embodiment is a strong or a weak basic resin. Examples of suitable strong or weak basic resins are described above.

In one embodiment of the invention, phosphate is precipitated from the eluate using at least one of MgCl₂, MgO and Mg(OH)₂ to obtain struvite.

In one embodiment, the process of the invention includes a step wherein the juice stream is subjected to cation exchange prior to or without fermentation of the juice stream. Cation exchange of the untreated juice at neutral pH, i.e. pH 6.0 to 8.0 or around pH 7.0, e.g. pH 6.5, may be used to recover basic amino acids like lysine and arginine, as well as cations like potassium. A suitable weak cation exchanger for use in this embodiment is a weak or strong acidic cation exchanger. A weak acidic cation exchanger is e.g. a resin comprising carboxylic acid groups. Suitable commercialy available examples of weak acidic cation exchangers include e.g. DOW MAC-3, Sybron CNN, Purolite C-104. A strong acidic cation exchanger is e.g. a resin comprising sulfonic acid groups, such as e.g. sodium polystyrene sulfonate or poly(2-acrylamido-2-methyl-1-propanesulfonic acid) (polyAMPS). Suitable commercialy available examples of strong acidic cation exchangers include e.g. DOW Marathon C10, Purolite PFC-100X10, Sybron CFP-110.

In one embodiment, the process of the invention includes a step wherein, the juice stream is subj ected to anion exchange at alkaline pH prior to or without fermentation of the juice stream. Anion exchange of the untreated juice at alkaline pH, i.e. a pH higher than pH 8.0, 8.5, 9.0, 9.5. or 10.0 may be used to recover most amino acids except for the basic amino acids lysine and arginine. The pH of the juice stream may be increased with any suitable base, e.g. sodium hydroxide or ammonia, of which gaseous ammonia is more preferred. Most preferred is gaseous ammonia obtained from calcium hydroxide treatment of a lignocellulosic material as herein defined below. A suitable weak anion exchanger for use in this embodiment is a weak basic resin. Examples of suitable weak basic resins are described above.

The unfermented juice stream subjected to one or more ion exchange steps for recovery of *inter alia* amino acids as described above, can subsequently be fed to a fermentation for conversion of the organic compounds in the juice stream to organic acids, such as lactic acid, as herein described above.

In a further preferred embodiment, the process of the invention includes a step wherein, the juice stream is subjected to a step wherein protein is removed from the juice stream by at least one of heat and acid coagulation and precipitation. Preferably, protein is removed from the juice stream prior to and/or without fermentation of the juice stream. Preferably also, protein is removed from the juice stream prior to ion exchange. Methods for heat and/or acid coagulation of proteins are well known in the art perse.

In a third aspect the invention relates to a process for recovery of components from a juice stream using ion exchange, such as the processes of the first and second aspects described herein above, and wherein the ions are eluted from the ion exchanger using ammonia. Preferably, the process comprises a step wherein anions are eluted from an anion exchanger using ammonia.

The ammonia used for elution from and regeneration of the ion exchangers in the processes of the invention, as well as the ammonia used to increase the pH of the juice stream in some of the embodiments of the invention, preferably is gaseous ammonia. Gaseous ammonia can be flushed from below through an ion exchange bed, e.g. in an essentially vertical column as described above, to create a sort of fluidised bed. Elution can thus take place in a minimal liquid volume and without increasing the elution volume, thereby allowing to obtain the eluted ions in highly concentrated eluate solutions.

The skilled person will appreciate that during elution with ammonia the pH will (gradually) change, i.e. increase. This effect can be used for successive recovery in separate fractions of components each eluting at a given pH or pH range. This may advantageously be used for recovery of various amino acids in separate fractions according to their isoelectric points.

In a preferred embodiments of the invention, the ammonia used for elution from and regeneration of the ion exchangers in the processes of the invention, as well as the ammonia used to increase the pH of the juice stream in some of the embodiments of the invention, is ammonia that is obtained from calcium hydroxide treatment of a lignocellulosic material, e.g. a biogas fermentation digestate, wastewater treatment sludge or manure.

Calcium hydroxide may be used in accordance with US 5,693,296 for pre-treatment or treatment of lignocellulosic materials such as e.g. a biogas fermentation digestate. Thus, lignocellulosic material to be treated can be fed into a chipper, grinder, chopper, shredder or the like, to be reduced in size. Resulting lignocellulosic material particles are then combined with calcium hydroxide and water to form an alkaline lignocellulosic material mixture. The mixture contains between about 2 to about 50 grams of water per gram of dry lignocellulosic material and preferably about 5 - 25 grams of water per gram of dry lignocellulosic material. The mixture also contains between about 2 to about 50 grams of calcium hydroxide per 100 grams of dry lignocellulosic material and preferably contains about 30 grams of calcium hydroxide per 100 grams of dry lignocellulosic material. Depending on the type of lignocellulosic material, the preferable amount may be more or less. Calcium hydroxide may be added before or after the water or as an aqueous solution or dispersion. The aqueous calcium hydroxide/lignocellulosic material mixture is maintained in reaction chambers, which are preferably stainless steel, at between about 40 °C to about 150 °C, preferably between about 80 °C to about 100 °C, and more preferably at about 95°C. Temperatures not exceeding 100 °C are preferred to avoid the need for pressurised equipment. Depending on the type of lignocellulosic material, the temperature range may be between about 70 °C to about 110 °C, between about 110 °C to about 150 °C, or between about 50 °C to about 65 °C. The temperature is maintained for between about 1 to about 36 hours, preferably between about 1 to about 20 hours, more preferably about 3 hours. Again, depending on the type of lignocellulosic material and the treatment temperature, the time period may be longer or shorter such as between about 15 to about 25 hours, but also 1 week, 2 weeks, 3 weeks and also several months.

The calcium hydroxide renders the lignocellulosic material more accessible, e.g. more amenable to enzymatic digestion, such that it may be used in a further round of biogas fermentation. Calcium hydroxide treatment of lignocellulosic material produces ammonia, which usually is recovered in acid washings using (diluted) sulphuric acid. In the third aspect of the invention, however, the process wherein ammonia is produced from the calcium hydroxide treatment of lignocellulosic material is advantageously integrated with ion exchange as practiced in the processes of the first and second aspects of the present invention. Thus, preferably the ammonia produced in the calcium hydroxide treatment of lignocellulosic material is collected, and optionally stored, and then used for elution and regeneration of the ion exchanger as described above. Integration of ammonia production from calcium hydroxide treatment of lignocellulosic material, preferably biogas digestate, with the ion exchange processes of the invention advantageously avoids the costs of acid washings and an ammonium sulphate waste stream and further enables regeneration of the ion exchangers without further costs for chemicals and avoiding generation of waste streams.

In a preferred embodiment, the process of 1) producing ammonia from calcium hydroxide treatment of lignocellulosic material and 2) the ion exchange processes are integrated in a manner that minimises transport between the two types of processes. Preferably therefore, ammonia production from calcium hydroxide treatment of lignocellulosic material, from e.g. a biogas fermentation is performed at a site that is 50, 20,10, 5, 2, 1, 0.5, 0.1, 0.05 or 0.01 km or less from the site where the ion exchange is performed.

In a further embodiment, the treated juice stream, i.e. the stream that is obtained after fermentation and ion exchange for recovery of the various components as described above, is fed into a biogas fermentation so as to minimise waste and environmental burden.

A process comprising all three aspects of the invention can e.g. be applied on a juice stream that is obtained in a grass-refining process. In such a process the grass is washed and then broken up and comminuted. The fibres separated from the juice stream and may e.g. be used for the production of paper or as material for insulation. The juice is subsequently heated to precipitate most of the proteins in the juice, which may then be separated using a decanter. The thus obtained juice stream may be further processed in the above described processes of the invention whereby valuable components are recovered from the juice stream in concentrated form using ion exchange and whereby sugars in the juice stream are fermented to lactic acid that is subsequently also recovered using ion exchange and elution with ammonia from biogas as described above, to yield ammonium lactate in concentrated form. In such a process a biogas fermentation plant of 0.1 - 50 MW may be combined with a grass-refining plant that processes about 1000 - 2000 ha (hectare, i.e. one square hectometre) or even larger, e.g. up to 5000, 10.000 or 20.000 ha or larger.

Components recovered in the processes of the invention may e.g. be applied in animal feed. A mixture of lactic acid and amino acids in their ammonium salts could e.g. be applied in animal feed. The ammonium could be replaced by another cation, such as calcium, for monogastric animals such as porcine and poultry, also to produce less of a burden on the environment. The ammonia thus obtained could then be applied in some other process where it is useful, thus avoiding its release into the environment.

Lactic acid recovered in the processes of the invention may be used as food and/or feed ingredient, as intermediate (precursor) for the production of the bioplastic polylactic acid (PLA), as a conservative of proteins, or to conserve grass or (wet) fibre e.g. for making paper.

Organic acid recovered from the juice stream, such as citric, fumaric or malic acid may also be applied in animal feed, or when fractionated from lactic acid be applied in human food. These acids and other organic acids like itaconic acid can be used as precursor in chemical synthesis.

Amino acids recovered in the processes of the invention may e.g. be not only applied in food and/or animal feed but also in pharmaceutical applications and/or as precursor in chemical synthesis. Amino acids recovered in the processes of the invention may advantageously be used to replace synthetic chemicals.

The concentrated phosphate obtained in the processes of the invention may be applied as fertiliser.

In one example of a process of the invention a combination of organic acid (such as e.g. lactate), phosphate and amino acids is obtained from the juice, while other less valuable components are disposed off, used or kept locally.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

### Examples

### Example 1: Recovery of lactic acid by ammonia elution

First to obtain a representative Lactic Acid Solution (LAS), grass juice was prepared in a lab scale blender/press and heated to 95 °C to precipitate most proteins. Then cooled and kept at 50 °C in a jacketed vessel for 1 hour, with the addition of Lactobacillus, to ferment the sugars to lactic acid. During fermentation, the pH was kept above 5, by addition of NaOH.
1.5 litre LAS was prepared as described above, and filtered. Then fed to the bottom of a column, filled with 100 gram of Amberlyst A21 a weak anion ion exchange resin. Due to the upward direction of the flow, resin was mixed throughout the column and acted more as a mixed system than as a plug flow system. The resin was kept in the column with a screen at the top, LAS flow rate of 10 ml/min.
After 1 hour, the LAS flow was stopped (but not drained) and the column fed with NH₃(g) (R717 from British Oxygen Company) at about 500 ml/min, for 15 minutes. At this rate, no gas escaped the column (confirmed by visual inspection only). The column was then drained to collect about 110 ml ammoniumlactate solution, a first sample (sample 1) was taken. Then the column was filled with 100 ml water, and drained again: sample 2. And filled with 100 ml water and drained again: sample 3.

Next, the column was filled with 100 ml water, and fed with NH₃(g) at 500 ml/min for another 15 minutes. The column was drained to collect ammoniumlactate: sample 4.

All four samples where analyzed for lactate content by HPLC method (with Aminex A6 column with 0.01N H₂SO₄ as eluent (1 ml/min) and UV detector, 214 nm). The results are shown in Table 1.

**Table 1 Lactate concentrations in subsequent eluate samples of Example 1.**

| **Eluate** | **Lactate concentrations (g/l)** |
|---|---|
| Sample 1 | 125 |
| Sample 2 | 20 |
| Sample 3 | 5 |
| Sample 4 | 55 |

### Example 2: Recovery of amino acids from juice from grass and algae

A combined stream of washed grass and fresh algae where fed to a Sprout-Waldron 12 inch pressure refiner (with grinding disks of the type D2A505) to break up the cells. The fresh algae stream was both used for providing plant mass and for cooling the refiner. After a pressing step, the juice was heated to 95 °C and brought to pH 5.7 by adding lactic acid. After separating the coagulated protein from the juice by a centrifuge, the juice was cooled to 30 °C. A weak anion exchanger, Amberlyst A21, was used to bind amino acids. First, the pH was increased to 6.5, by addition of ammonia. 400 ml of this solution was mixed with 150 g Amberlyst A21, and stirred for one hour at 30 °C. The resin was removed from the mixture with a screen and stored for further analysis. The remaining solution was brought to pH 9 by addition of ammonia, mixed with a second load of 150 g resin, and stirred for one hour at room temperature. Again, the resin was separated from the solution and kept for further analysis.

The experiment was repeated with a strong resin, Amberlite IRA400, to obtain two more samples of loaded resin. First, the resin was regenerated by adding it to 220 ml 1M NaOH. 100 ml was used to determine overall nitrogen content. Another 100 ml was used to measure individual amino acids. The results are shown in Table 2 from which it is clear that Amberlyst A21 binds less of the remaining amino acids at pH 9. Amino acid analysis by HPLC showed that at pH 6.5 most of the total Glu and Asp is captured. At pH 9, Met, Cys, Tyr, His, Phe, Trp, Ser, Thr, Asn, and Gln were found.

**Table 2 Overall nitrogen content measurements of eluates**

| | Nitrogen content (Kjeldahl) in g/100ml) | |
|---|---|---|
| Type ion exchanger | Amberlyst A21 | Amberlite IRA400 |
| Sample pH 6.5 | 0.83 | 0.90 |
| Sample pH 9 | 0.03 | 0.71 |

### Example 3: Isolation of organic acids from a biogas co-fermentation on corn and animal manure.

Carbohydrates present in a biogas fermentation known in the art as described by e.g. Appels et al (2008, Progress in Energy and Combustion Science, Volume 34, Issue 6, December 2008, Pages 755-781, ISSN 0360-1285, DOI: 10.1016/j.pecs.2008.06.002. (http://www.sciencedirect.com/science/article/B6V3W-4T5JP78-1/2/745cc5ac970993d5a2a9146c78420e54) are converted by the microbial consortia present into organic acids like acetic acid, propionic acid and butyric acid. After inocculation of 10 liters of pig manure containing 1 kg of the full chopped corn plant, half liter samples were taken after 3 and 6 days of incubation. These were centrifuged a 3000 rpm for 20 minutes. The supernatants were incubated with a strong anion exchange resin, Amberlyst A21 The loaded resins were washed in a column against gravity with 1 liter of water, pH 7. Then the two resins were eluted by 100 ml of 3 M NaOH and rinsed with another 100 ml of water. The organic acids were analysed by HPLC with an Aminex HPX-87 column with 0.013M H₂SO₄ as mobile phase. Results are shown in Table 3.

**Table 3: Organic acids obtained from a biogas co-fermentation on corn and animal manure.**

| | Organic acid content in the eluent (g) | |
|---|---|---|
| Fermentation time | 3 days | 6 days |
| Acetic | 5.2 | 4.3 |
| Propionic | 3.4 | 3.2 |
| Butyric | 1.2 | 1.9 |

### Example 4: Production of NH₄NO₃ fertilizer from manure

10 kg of cattle manure and 20 liters of water was fermented to form nitrate by nitrifying bacteria. After 5 days a sample of 0.5 liter is taken and centrifuged in a sorvall centrifuge at 3000 rpm for 30 minutes. The supernatant is mixed with 200 grams of a weak anion exchange resin, Amberlite IRA400. The resin is washed with 1 liter of water against gravity and then ammonia (gas) is bubbled through the column. After elution with 200 ml of water 21 gram of ammonium nitrate was detected in the water.

### Example 5: Recovery of organic acids from cellulose acid pre-treatment

20 gram of wheat straw is incubated in 100 ml of 50 mM fumaric acid for 30 minutes at 150 °C. After cooling down, the sample is washed by 250 ml water on a Buchner filter. This eluate is passed on 20 ml column of a strong ion exchange resin (Amberlyst A21). Then Ammonia gas is bubbled through the column. The column is eluted with 25 ml of water and the amount of fumarate was determined by HPLC 3.4 gram.

## Claims

1. A process for recovery of components from a bio-organic juice stream, the process comprising the steps of: a) fermenting organic compounds in the juice stream to organic acids; and b) recovery of the organic acids from the fermented juice stream using anion exchange.

2. A process according to claim 1, wherein the bio-organic juice stream is a vegetable juice stream.

3. A process according to claim 1 or 2, wherein organic compounds in the juice stream are present at a concentration of no more 5% (w/v).

4. A process according to claim 3, wherein the organic compounds are fermented to organic acids comprising at least lactic acid.

5. A process according to any one of claims 1 - 4, wherein the fermentation is an anaerobic fermentation.

6. A process according to any one of claims 1 - 5, wherein the fermentation temperature is higher than about 42°C.

7. A process according to any one of claims 1 - 6, wherein organic acids, amino acids and other anions in the fermented juice stream are recovered by anion exchange without prior neutralisation of pH.

8. A process according to any one of claims 1 - 7, wherein the organic acids, amino acids and other anions in the fermented juice stream are recovered by anion exchange after increasing the pH of the fermented juice stream.

9. A process according to any one of claims 1 - 8, wherein prior to the fermentation of the juice stream, the juice stream is subjected to anion exchange at neutral or acidic pH for recovery of organic acids, amino acids and other anions.

10. A process according to any one of claims 1 - 9, wherein prior to the fermentation of the juice stream, the juice stream is subjected to cation exchange.

11. A process according to any one of claims 1 - 10, wherein anions are eluted from the anion exchanger using ammonia.

12. A process according to claim 11, wherein the ammonia is obtained from calcium hydroxide treatment of lignocellulosic material.

13. A process according to claim 12, wherein the lignocellulosic material is at least one of a biogas fermentation digestate, waste water sludge and manure.

14. A process according to claim 12 or 13, wherein the recovery of anions from the juice stream is performed at a site that is 10 km or less from the site where the lignocellulosic material is treated.

15. A process according to any one of claims 1 - 14, wherein prior to ion exchange, protein is removed from the juice stream by at least one of heat coagulation and acid coagulation, and optionally decantation.
